# EUROPEAN PATENT APPLICATION

(11) **EP 1 354 608 A1**
(43) Date of publication of application: **22.10.2003**
(21) Application number: 03076042.5
(22) Date of filing: 09.04.2003
(51) Int. Cl.: A61M 5/315, A61M 5/30, A61M 5/46, B05C 17/01

(54) **Jet injector having mechanism for creating air-in-tip**

(30) Priority: 15.04.2002 US 123898
(71) Applicant: Avant Drug Delivery Systems, Inc., San Diego, CA 92109 (US)
(72) Inventor: Slate, John B., San Diego, California 92109 (US); Burk, Michael W., San Marcos, California 92069 (US); Gorton, Lanny A., San Diego, California 92124 (US)
(74) Representative: Shortt, Peter Bernard

(57) **Abstract**

An injector for injecting a fluid medicament includes an injector body (20), a medicament chamber (34) and a plunger (36) that can be advanced into the chamber (34) to expel medicament from the distal end of the chamber. An air-in-tip mechanism is provided within the injector body for partially withdrawing the plunger (36) from the chamber (34) to create an air pocket (42) in the chamber adjacent the distal end. The air-in-tip mechanism includes a gripper ring (50) that is positioned within the injector body (20) and initially oriented to allow the plunger (36) to move freely through the gripper ring (50). A user operable button (52) is attached to the gripper ring (50) to first tilt the gripper ring (50) until it engages the plunger (36) and then move the plunger (36) proximally to create the air pocket (42). Alternatively, a face cam (72) and push rod (70) assembly can be activated when the injector's locking nut (132) is turned to automatically tilt and move the gripper ring (150).

## Description

### FIELD OF THE INVENTION

The present invention pertains generally to devices for injecting a fluid medicament into a patient. More particularly, the present invention pertains to needle-free (jet) injectors for infusing a fluid medicament into a patient. The present invention is particularly, but not exclusively, useful as a jet injector having a mechanism for creating an air pocket in the distal portion of the syringe prior to an injection.

### BACKGROUND OF THE INVENTION

Needle-free injectors have been used for many years for the purpose of infusing fluid medicaments into a patient. Indeed, they have several advantages over needle-type injectors. For instance, needleless injectors lend themselves to schedules where a large number of patients are to be inoculated at the same time. Most importantly, they do not incorporate sharp or pointed projections that can inadvertently stick into the care giver, or into some other third person. In recent years, the avoidance of so-called "sharps," that can cause inadvertent sticks, has been a design objective of many medical devices.

In their operation, all needle-free (jet) injectors rely on the generation of fluid pressures in the fluid medicament. Specifically, the purpose of generating these pressures is two-fold. First, it is necessary to create a hole in the skin of the patient. Second, it is necessary to thereafter maintain a substantially constant pressure for infusion of the fluid medicament into the patient through the hole. The magnitude and duration of these fluid pressures will, in large part, depend on the type of injection to be given.

There are basically three different types of injections that may need to be performed by a needleless injector. These are: 1) shallow, intra-dermal injections where the fluid medicament is infused directly into the skin; 2) medium depth, subcutaneous injections where the fluid medicament is infused into the fatty tissue beneath the skin; and 3) deeper intra-muscular injections where the fluid medicament is delivered directly into muscle tissue. Thus, depending on the type of injection that is desired, and the general nature or condition of the patient's skin, the fluid pressure that is necessary to make an appropriate hole can vary from injection to injection.

Insofar as needle-free injectors are concerned, the initial immediate rise of pressure in the fluid medicament that is necessary to create a hole in the skin of a patient is typically generated by propelling a drive bar into a syringe plunger. The resultant impulse force then causes a pressure rise in the fluid medicament. This, in turn, causes the fluid medicament to penetrate the skin, and thereby create the necessary hole for subsequent infusion. An example of such a device is provided in U.S. Patent No. 5,911,703 for an invention of Slate et al. that is entitled "Two-Stage Fluid Medicament Jet Injector" and that is assigned to the same assignee as the present invention.

Heretofore, when using needleless injectors, the practice has been to position a pre-filled injection tube directly against the skin of the patient. This, however, also places the fluid medicament that is in the tube in direct contact with the skin. Consequently, because the fluid medicament is already in contact with the skin, the impulse force that is created as the drive bar impacts the plunger is significantly attenuated by the time its effect is felt between the fluid medicament and the skin of the patient. An initial consequence of this is that the fluid medicament has insufficient momentum to penetrate the skin. Thus, it happens, at least initially, that the fluid medicament can seep around the injector and puddle on the surface of the skin. It would be desirable, however, to increase the momentum of the initial portion of medicament exiting the injector for the purpose of creating a hole in the skin of a patient. Preferably, this can be done without necessarily resorting to larger and faster drive bars, while also avoiding the seepage and puddling of the fluid medicament on the skin of the patient.

With needleless injectors there is always the requirement that a jet pressure be developed which is sufficient to cause the fluid medicament to penetrate the skin. One way to accomplish this is to use a heavy drive bar that will generate the necessary momentum. Heavy drive bars, however, also generate an undesirable recoil and, for spring-loaded injector mechanisms, a heavy drive bar will require a spring with a relatively large spring constant. Consequently, depending on the type of mechanism that is used to propel the drive bar, injector mechanisms that use springs having large spring constants to propel heavy drive bars can be hard to cock. Although lighter drive bars will overcome these undesirable consequences, lighter drive bars will necessarily have less momentum under the same circumstances.

One way to increase the momentum of the initial portion of medicament to exit the injector is to create an air pocket in the distal tip of the injection tube. An example of such a method is provided in co-pending U.S. Patent Application No. 09/665,849 filed September 20, 2000 for an invention of Slate et al. that is entitled "Air-in-tip Jet Injector" and that is assigned to the same assignee as the present invention. As disclosed therein, the creation of an air pocket in the distal tip of the injector tube can increase the initial jet pressures generated by the drive bar / plunger impact to between about twenty percent (20%) and five hundred percent (500%) above the pressures generated when no air pocket is present in the injection tube.

An effective way to create an air pocket in the tip of an injection tube is to withdraw the plunger from the medicament chamber prior to an injection. However, to obtain an appropriately sized air pocket (i.e. roughly 3 to 27 microliters) in a typical syringe, a plunger movement of only approximately 0.003 to 0.040 inches is required. Unfortunately, these small plunger movements are hard to achieve with any consistency using manual techniques (i.e. by manually withdrawing the plunger while eyeing the size of the resulting air pocket). This is especially true for people with impaired vision.

As indicated above, to effectively and consistently increase the momentum of the initial portion of medicament exiting the injector, an air pocket having a rather tightly controlled volume is generally required. This required air pocket volume, however, is also dependent on the amount of medicament in the syringe. In more detail, when a relatively high volume of medicament is in the syringe, the distance that the drive bar is allowed to accelerate is shorter. Because, the resulting impact force between the drive bar and plunger is smaller, the corresponding pressure increase in the medicament will also be smaller. It follows that a somewhat larger air pocket is required to provide the momentum increase necessary to adequately create a hole in the skin. To summarize, a larger air pocket volume is generally required when a relatively high volume of medicament is present in the syringe.

In light of the above, it is an object of the present invention to provide a jet injector having a mechanism for establishing an air pocket in the injection tube of the injector to thereby allow the initial portion of medicament ejected from the injector to exit the injector with sufficient momentum to create a hole in the skin of a patient. It is another object of the present invention to provide a mechanism for creating an air pocket in a jet injector that allows for penetration of the patient's skin with a relatively small injector driving force thereby minimizing recoil and reducing the force required to cock the injector. Another object of the present invention is to provide a mechanism for creating an air pocket in a jet injector that allows for injection of a fluid medicament into a patient that avoids the leakage or seepage of fluid medicament onto the skin of a patient. It is still another object of the present invention to provide a mechanism for a jet injector that reliably creates air pockets having accurate and consistent volumes. Still another object of the present invention is to provide a mechanism that automatically creates an air pocket when the user turns a locking ring to secure the syringe to the injector. Still another object of the present invention is to provide a mechanism for creating an air pocket in a jet injector that does not increase the size of the injector, uses a minimal number of parts and does not otherwise interfere with the operation of the injector. Another object of the present invention is to provide a mechanism that creates a relatively small air pocket in a syringe having a relatively low volume of medicament and a relatively large air pocket in a syringe having a relatively high volume of medicament. Yet another object of the present invention is to provide a mechanism for creating an air pocket in a jet injector that is dependable, simple to operate and comparatively cost effective.

### SUMMARY OF THE PREFERRED EMBODIMENTS

In accordance with the present invention, an injector for injecting a fluid medicament into a patient includes an injector body and a syringe. For the present invention, the syringe includes a fluid chamber and a plunger that can be advanced into, and withdrawn from, one end of the fluid chamber. At the other end of the fluid chamber, the syringe preferably includes a cone-shaped injection tube that extends from the fluid chamber and terminates at a distal port. During an injection, the plunger is advanced into the fluid chamber expelling fluid medicament from the chamber, through the injection tube and out of the distal port.

Prior to an injection, a medicament-filled syringe is loaded into the injector body. The injector body preferably includes a substantially cylindrical wall that is sized to receive and hold the syringe. As such, the cylindrical wall defines a longitudinal axis for the injector body. For the present invention, the injector body further includes a component for storing energy, such as a drive spring, that can be released to propel a drive bar into the syringe plunger. Importantly, a mechanism is provided within the injector body for partially withdrawing the plunger from the fluid chamber to create an air pocket within the injector tube (i.e. an air-in-tip mechanism).

A summary of the operation of the injector illustrates the role of the air-in-tip mechanism. First, a medicament-filled syringe is loaded into the injector body and secured. Next, the air-in-tip mechanism is activated to partially withdraw the plunger from the fluid chamber and create an air pocket within the injector tube and adjacent the distal port. With the air pocket created, the distal port of the injector is then held against the patient's skin and the injector is fired. Upon firing, the drive spring is released sending the drive bar into the plunger and expelling medicament through the injection tube and out the distal port. For the present invention, the air pocket provides a space for the initial portion of medicament to travel and gain momentum prior to contacting the patient's skin. The resulting momentum allows the medicament to create a hole in the skin for subsequent infusion of the remainder of the medicament into the patient.

In a first embodiment of the present invention, the air-in-tip mechanism includes a gripper ring that is formed with an aperture. The gripper ring is positioned within the cylindrical wall of the injector body and centered on the longitudinal axis. For the present invention, the gripper ring is initially positioned in a first orientation wherein the gripper ring is substantially perpendicular to the longitudinal axis. With the gripper ring in the first orientation, the syringe plunger is able to move freely through the aperture, and along the axis.

The air-in-tip mechanism further includes a button that is slideably mounted on the injector body and attached to the gripper ring at a first point on the periphery of the gripper ring. In greater detail, the button is moveable back and forth along a path that is parallel to the longitudinal axis. Initial movement of the button in the proximal direction reorients the gripper ring from the first orientation to a second orientation wherein the gripper ring is tilted relative to the axis. To tilt the gripper ring, a spring is provided which acts between the injector body and a second point on the gripper ring that is located on the gripper ring's periphery opposite the first point where the button is attached. With this cooperation of structure, the button moves the first point while the spring holds the second point to tilt the gripper ring relative to the axis. In the second orientation, the tilted gripper ring engages the syringe plunger causing the syringe plunger to move with the gripper ring. Subsequent movement of the button in the proximal direction overcomes the spring force and moves the entire gripper ring and plunger (in the proximal direction) to draw air into the injection tube and create an air pocket therein.

In accordance with the present invention, the air-in-tip mechanism further includes a stop to limit travel of the button in the proximal direction. Thus, the button moves through a predetermined distance that corresponds to a plunger movement that will create an air pocket having a desired volume. Once the button has been moved through this predetermined distance, a spring mounted between the button and the injector body returns the button to its initial position. During the return portion of the button cycle, the plunger remains stationary. More specifically, initial movement of the button in the distal direction causes the gripper ring to return to the first orientation wherein the gripper ring is substantially perpendicular to the longitudinal axis. With the gripper ring in the first orientation, the gripper ring moves freely over the syringe plunger. Subsequent movement of the button in the distal direction causes the gripper ring to return to its original position while the syringe plunger remains stationary. With the gripper ring at its original position and in the first orientation, the injector is ready for firing. Since the gripper ring is in the first orientation, the gripper ring does not affect plunger movement during the injection.

In another embodiment of the present invention, the air-in-tip mechanism is activated automatically when the syringe is loaded into the injection body and secured (i.e. a user operated button is not required in this embodiment). Like the embodiment described above, in this embodiment a gripper ring is used for engaging and moving the syringe plunger to create the air pocket in the injection tube. To tilt and axially move the gripper ring, the air-in-tip mechanism includes a push rod and a face cam that is mounted on the locking ring of the injector. As a medicament-filled syringe is initially inserted into the injector body, the plunger of the syringe is received through the aperture of the gripper ring. The syringe is then securely engaged with the injector body by rotating the locking ring. With this rotation, the face cam that is mounted on the locking ring rotates with the locking ring.

The push rod, which is slideably mounted on the injector body, has one end that rests on the cam surface of the face cam. The opposite end of the push rod urges against a peripheral tab on the gripper. Accordingly, as the face cam is rotated with the locking ring, it advances the push rod in a proximal direction moving the peripheral tab on the gripper ring. This movement tilts the gripper ring into its engagement with the syringe plunger. Once the gripper ring has been engaged with the plunger, any additional proximal advancement of the push rod causes the plunger to retract or withdraw from the fluid chamber of the syringe. This withdrawal then draws air into the injection tube.

In either embodiment described above, a tapered plunger can be used to vary the amount of air that is drawn into the injection tube in proportion to the volume of fluid medicament that is initially present in the syringe. Specifically, for this purpose, the plunger is configured with a taper of decreasing diameter in the proximal direction. The consequence of this configuration is best appreciated by comparing the case where there is a relatively large volume of fluid in the syringe with the case where there is a relatively small volume of fluid in the syringe.

The volume of fluid that is in the syringe will determine the position of the plunger. Specifically, the more fluid there is in the syringe, the more proximal will be the position of the plunger. Consequently, with more fluid in the syringe, a larger diameter portion of the plunger will be disposed within the aperture of the gripper ring. Thus, during tilting of the gripper ring, the gripper ring will engage the large diameter portion of the plunger earlier and retract the plunger through a greater distance. Accordingly, with more fluid in the syringe, more air will be drawn into the injection tube.

### BRIEF DESCRIPTION OF THE DRAWINGS

The novel features of this invention, as well as the invention itself, both as to its structure and its operation, will be best understood from the accompanying drawings, taken in conjunction with the accompanying description, in which similar reference characters refer to similar parts, and in which:
Fig. 1 is an elevational view of an injector body in accordance with the present invention;
Fig. 2 is a sectional view of the injector body shown in Fig. 1, shown after a syringe has been installed in the injector body, with portions shown in cross-section, as seen along the line 2-2 in Fig. 1, for clarity;
Fig. 3 is a sectional view of the distal portion of a syringe as would be seen along line 2-2 in Fig. 1, shown after an air pocket has been created in the tip of the injection tube;
Fig. 4 is an enlarged, detail view as seen along line 4-4 in Fig. 2 showing an air-in-tip mechanism for creating an air pocket in the tip of the injector tube with the mechanism shown prior to activation;
Fig. 5 is an enlarged, detail view as in Fig. 4 of the air-in-tip mechanism during activation showing the gripper ring of the mechanism engaging the syringe plunger;
Fig. 6 is an enlarged, detail view as in Fig. 4 of the air-in-tip mechanism during activation showing the position of the syringe plunger after the gripper ring has engaged the plunger and moved the plunger proximally to create an air pocket;
Fig. 7 is an enlarged, detail view as in Fig. 4 of the air-in-tip mechanism after activation showing the new position of the syringe plunger after the gripper ring has engaged the plunger, moved the plunger proximally and then returned to its original position;
Fig. 8 is a sectional view as in Fig. 2 of an alternate embodiment of the present invention in which a face cam and push rod are used in conjunction with the gripper ring to reposition the syringe plunger;
Fig. 9 is a perspective view of the gripper ring shown in Fig. 8;
Fig. 10 is a perspective view of the face cam shown in Fig. 8 with portions removed to show the cam surface of the face cam;
Fig. 11 is an enlarged, detail view as in Fig. 5 of an alternate embodiment of the present invention wherein the syringe plunger is formed with a taper with the plunger shown positioned in a syringe containing a relatively large amount of medicament; and
Fig. 12 is an enlarged, detail view as in Fig. 11 showing a tapered plunger positioned in a syringe containing a relatively small amount of medicament.

### DESCRIPTION OF THE PREFERRED EMBODIMENT

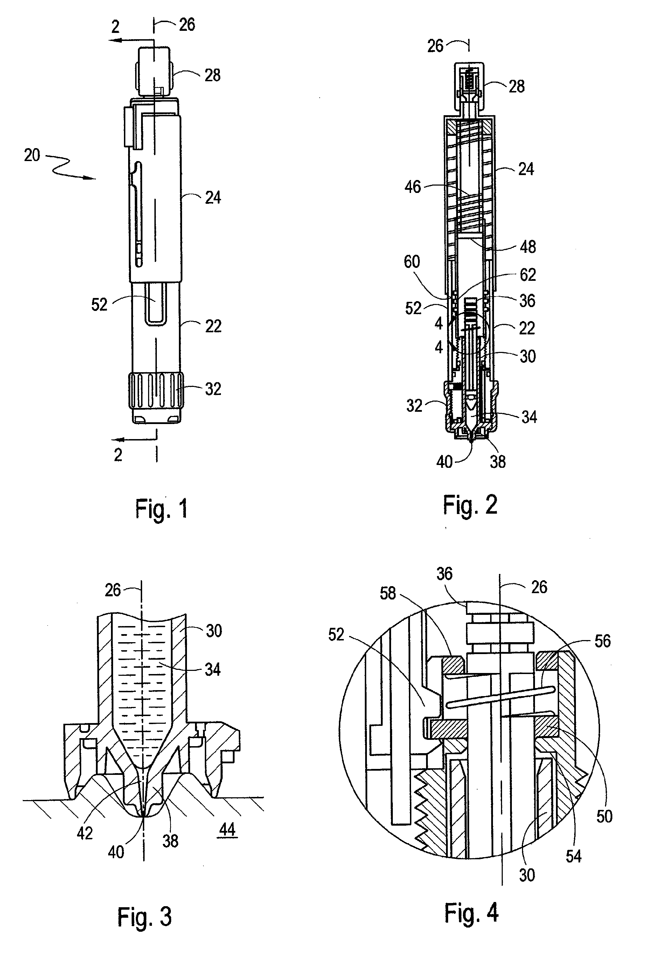

Referring initially to Fig. 1, an exemplary injector body in accordance with the present invention is shown and is generally designated 20. As shown, the injector body 20 includes a distal tube 22 and a proximal tube 24. For the present invention, the distal tube 22 and proximal tube 24 are substantially cylindrical and centered on a longitudinal axis 26. Fig. 1 further shows that the distal tube 22 is preferably smaller in diameter than the proximal tube 24, allowing the distal tube 22 to be partially disposed within the proximal tube 24. Also shown, a firing cap 28 is provided at the proximal end of the injector body 20 that can be depressed to initiate an injection.

Referring now to Fig. 2, it can be seen that a syringe 30 can be disposed in the distal tube 22 and secured to the distal tube 22 by a locking ring 32. As further shown in Fig. 2, the syringe 30 includes a fluid chamber 34 and a plunger 36 that can be advanced into the fluid chamber 34 or, alternatively, withdrawn from the fluid chamber 34. Also, the syringe 30 is formed with an injection tube 38 that extends from the fluid chamber 34 and terminates at a distal port 40. Importantly, the injection tube 38 establishes fluid communication between the fluid chamber 34 and the distal port 40. Further, and also importantly, the injection tube 38 is formed with a taper of decreasing cross section in a distal direction. Stated differently, the injection tube 38 decreases in diameter in the distal direction (the distal direction is from the fluid chamber 34 toward the distal port 40). The exact configuration of the injection tube 38 for the syringe 30 is somewhat a matter of design choice. It is, however, preferable that the surface transition in the injection tube 38 between the fluid chamber 34 and the distal port 40 be smooth, and have a minimum of irregularities, such as so-called "shoulders."

To prepare the syringe 30 for an injection, an air pocket 42 is first created in the injection tube 38, adjacent the distal port 40 as shown in Fig. 3. In accordance with the present invention, the injection tube 38 and fluid chamber 34 are first filled with a fluid medicament and the fluid filled syringe 30 is then loaded into the injector body 20 (shown in Fig. 2). After the injector body 20 has been loaded with the filled syringe 30, an air-in-tip mechanism (described in detail below) is activated to partially retract the plunger 36 from the fluid chamber 34 and draw air through the distal port 40 to create the air pocket 42.

With the air pocket 42 created, the distal port 40 of the syringe 30 is then held against the patient's skin 44 and the plunger 36 is rapidly advanced into the fluid chamber 34. For this purpose, as shown in Fig. 2, a drive spring 46 can be used to propel a drive bar 48 into the plunger 36. It is to be appreciated that other means well known in the pertinent art for rapidly advancing a plunger 36 into the fluid chamber 34 can be used in the present invention in place of the drive spring 46 / drive bar 48 assembly. In any case, as the plunger 36 is advanced into the fluid chamber 34, the fluid medicament is urged from the fluid chamber 34 and through the injection tube 38. Due to the air pocket 42, however, there is little, if any, resistance to the movement of the fluid medicament through the injection tube 38. Thus, at least initially, the fluid medicament is accelerated as it passes through the injection tube 38. In doing so, the fluid medicament gains momentum. The consequence of the fluid medicament gaining momentum as it passes through the space in the injection tube 38 that is created by the air pocket 42 is that the initial portion of medicament exiting the distal port 40 will have sufficient momentum to create a hole in the skin 44. With the hole established in the skin 44, the remainder of the fluid medicament in the syringe 30 can be infused into the patient through the hole in the skin 44.

In accordance with the present invention, the size of the air pocket 42 can be varied to accommodate different types of injections as well as variations in the condition and nature of the patient's skin where the injection is to be made. For example, for an intra-dermal injection where the depth of penetration is minimal, the air pocket 42 will preferably be small. As the depth of penetration for the fluid medicament is increased (e.g. subcutaneous and intra-muscular injections), the size of the air pocket 42 will be proportionately increased. Also, when considering skin condition, it will be appreciated that as a patient's skin is thicker and tougher it may be desirable or necessary to increase the size of the air pocket 42. For most applications, the size or volume of the air pocket 42 will be in a range between approximately three and twenty-seven microliters (3-27 µl) and is more preferably in a range between approximately five and eight microliters (5-8 µl).

Referring now with cross reference to Figs. 2 and 4, an air-in-tip mechanism is shown for partially retracting the plunger 36 from the fluid chamber 34 to create an air pocket 42 (see Fig. 3). In accordance with the present invention, the air-in-tip mechanism includes a gripper ring 50 that is formed with an aperture. As shown, the gripper ring 50 is positioned within the distal tube 22 and substantially centered on the longitudinal axis 26. The air-in-tip mechanism further includes a button 52 that is slideably mounted on the distal tube 22 of the injector body 20 and attached to the gripper ring 50 at a first point on the periphery of the gripper ring 50. With this cooperation of structure, the button 52 is moveable back and forth along a path that is parallel to the longitudinal axis 26. As shown in Fig. 1, the button 52 is accessible from the outside of the injector body 20 to allow the user to operate the button 52 after the injector body 20 has been loaded with a syringe 30.

With continued cross reference to Figs. 2 and 4, is can be seen that the distal tube 22 is formed with a plunger guide 54 having a beveled edge to align the plunger 36 along the axis 26 when the injector body 20 is loaded with a syringe 30. The air-in-tip mechanism further includes a gripper return spring 56 for urging the gripper ring 50 against the plunger guide 54. In greater detail, the return spring 56 is disposed between the gripper ring 50 and an abutment 58 formed in the distal tube 22. It can also be seen that the return spring 56 acts between the abutment 58 and a second point on the gripper ring 50 that is located on the periphery of the gripper ring 50 opposite the first point where the button 52 is attached.

Figs. 4 through 7 illustrate the sequential operation of the air-in-tip mechanism. Beginning with Fig. 4, it can be seen that the gripper ring 50 is initially positioned in a first orientation wherein the gripper ring 50 is substantially perpendicular to the longitudinal axis 26. With the gripper ring 50 in the first orientation, the plunger 36 is able to move freely through the aperture of the gripper ring 50, and along the axis 26. Thus, when the syringe 30 is initially loaded into the injector body 20, the plunger 36 passes freely through both the plunger guide 54 and the aperture of the gripper ring 50, and becomes positioned relative to the gripper ring 50 and distal tube 22 as shown in Fig. 4.

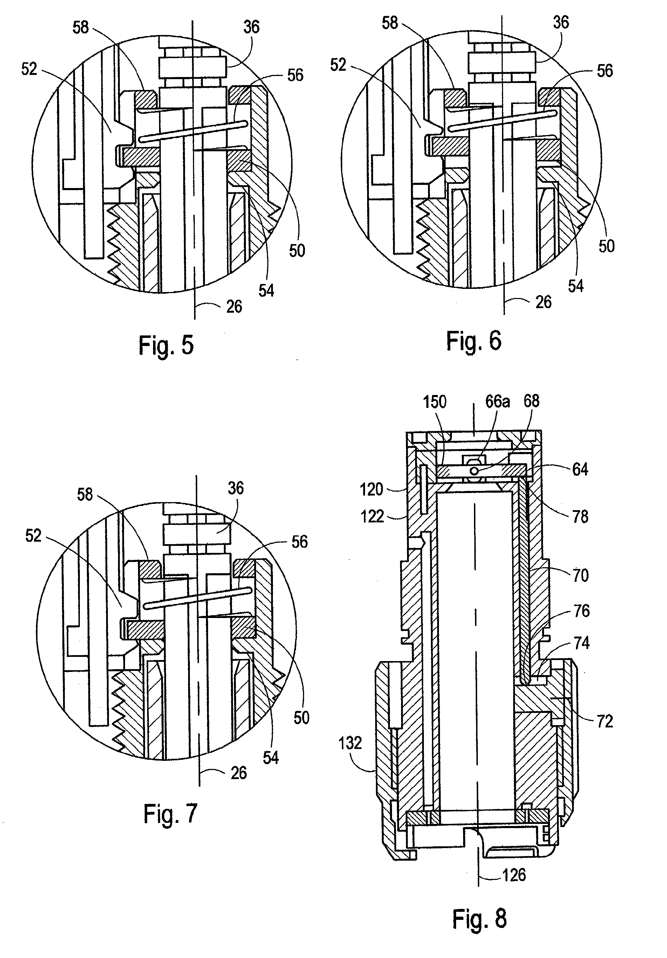

Fig. 5 shows the configuration of the air-in-tip mechanism after initial movement of the button 52 in the proximal direction. As shown, initial movement of the button 52 reorients the gripper ring 50 from the first orientation to a second orientation wherein the gripper ring 50 is tilted relative to the axis 26. Tilting of the gripper ring 50 is achieved because the button 52 moves the point on the periphery of the gripper ring 50 where the button 52 is attached, while the return spring 56 holds the point located on the periphery of the gripper ring 50 opposite the point where the button 52 is attached. In the second orientation, the tilted gripper ring 50 engages the plunger 36 causing the plunger 36 to move with the gripper ring 50.

Referring now to Fig. 6, it can be seen that subsequent movement of the button 52 in the proximal direction overcomes the resistance of the return spring 56 and moves the entire gripper ring 50 and plunger 36 (in the proximal direction) to draw air into the injection tube 38 (see Fig. 3) and create an air pocket 42 therein. As shown in Fig. 2, the air-in-tip mechanism further includes a stop 60 to limit travel of the button 52 in the proximal direction. It is to be appreciated that the volume of the air pocket 42 can be altered by adjusting the position of stop 60. Thus the button 52 moves through a predetermined distance that causes the plunger 36 to move through a distance that will create an air pocket 42 having a desired volume. For most applications, the size or volume of the air pocket 42 will be in a range between approximately three and twenty-seven microliters (3-27 µl) which corresponds to a movement of the plunger 36 through a distance in the range of approximately 0.003 inches to 0.040 inches. To target a more preferable volume of the air pocket 42 in the range between approximately five and eight microliters (5-8 µl), a movement of the plunger 36 through a distance of approximately 0.008 inches ± 0.003 inches is set.

Fig. 2 also shows that a spring 62 is mounted between the button 52 and the distal tube 22 to return the button 52 to its initial position after the operator has moved the button 52 through the predetermined distance. With cross-reference to Figs. 6 and 7, it can be seen that the plunger 36 remains stationary while the button 52 and gripper ring 50 return to their initial positions. More specifically, after the user has moved the button 52 through the predetermined distance and the button 52 has contacted the stop 60, initial movement of the button 52 in the distal direction causes the gripper ring 50 to return to the first orientation wherein the gripper ring 50 is substantially perpendicular to the longitudinal axis 26. With the gripper ring 50 in the first orientation, the gripper ring 50 moves freely over the plunger 36. Thus, movement of the button 52 in the distal direction causes the gripper ring 50 to return to its original position while the plunger 36 remains stationary (in its retracted position). With the gripper ring 50 at its original position and in the first orientation, the air-in-tip mechanism is configured for an injection. As shown in Fig. 7, with the gripper ring 50 in the first orientation, the gripper ring 50 does not affect movement of the plunger 36 during an injection.

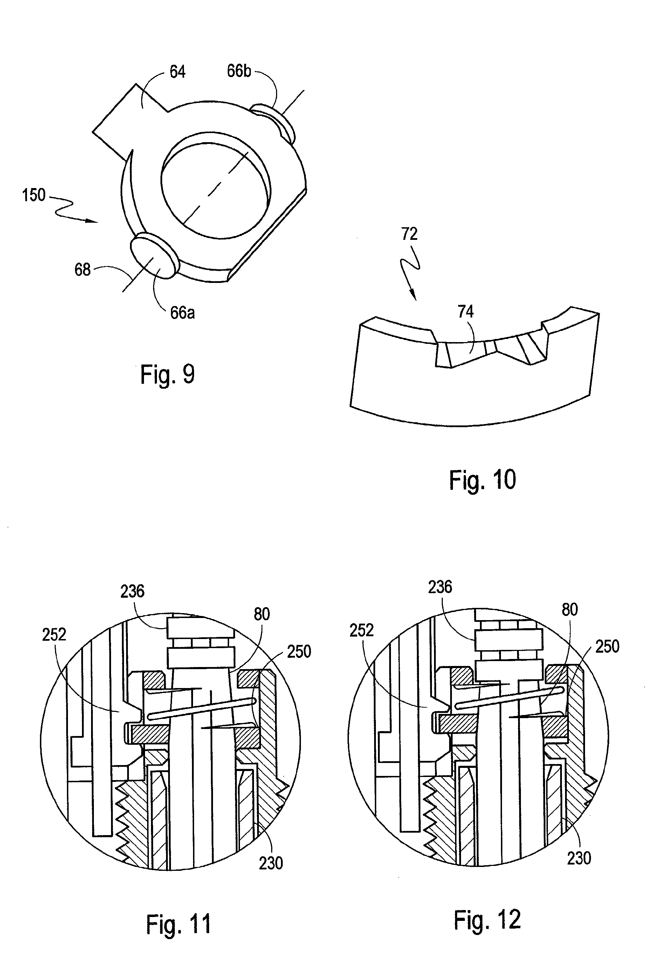

Fig. 8 shows another embodiment of an air-in-tip mechanism in accordance with the present invention. In this embodiment, the air-in-tip mechanism is activated automatically when a syringe (such as syringe 30 having plunger 36 shown in Fig. 2) is loaded into the injection body 120 and secured. Like the embodiment described above, in this embodiment a gripper ring 150 is used for engaging and moving the plunger 36 to create the air pocket 42 in the injection tube 38 (see Fig. 3). A better appreciation of the gripper ring 150 can be obtained with reference to Fig. 9. As shown, the gripper ring 150 is substantially shaped as a ring surrounding an aperture and is formed with a tab 64 along a portion of the periphery of the ring. The gripper ring 150 is further formed with a pair of cylindrically shaped mounting pads 66a,b with the first mounting pad 66a being positioned opposite the second mounting pad 66b along the periphery of the ring. As further shown, the opposed mounting pads 66a,b define a pivot axis 68 for the gripper ring 150.

Returning now to Fig. 8, it can be seen that the gripper ring 150 is positioned in the distal tube 122 and pivotally mounted to the distal tube 122. More specifically, the mounting pads 66 are fastened to the distal tube 122 to allow the gripper ring 150 to pivot about the pivot axis 68. As further shown in Fig. 8, the air-in-tip mechanism includes a face cam 72 that is mounted on the locking ring 132 and a push rod 70. As best seen with cross reference to Fig. 10, the face cam 72 is formed with a cam surface 74 having a raised midpoint (i.e. a cam). Also shown in Fig. 8, the locking ring 132 is rotatably mounted on the distal tube 122 for rotational movement together with the face cam 72 about the longitudinal axis 126 of the distal tube 122. With this cooperation of structure, a medicament-filled syringe 30 having a plunger 36 (see Fig. 2) can be loaded into the injector body 120. During loading, the plunger 36 of the syringe 30 is received through the aperture of the gripper ring 150. The syringe 30 is then secured to the injector body 120 by rotating the locking ring 132 about the longitudinal axis 126. With this rotation, the face cam 72 that is mounted on the locking ring 132 is also rotated about the longitudinal axis 126.

With continued reference to Fig. 8, it can be seen that the push rod 70 is positioned in a passageway formed in the distal tube 122. It can be further seen that one end 76 of the push rod 70 rests on the cam surface 74 of the face cam 72 and the other end 78 of the push rod 70 urges against the tab 64 of the gripper ring 150. With this cooperation of structure, the push rod 70 is advanced in the proximal direction in response to a rotation of the face cam 72 and locking ring 132. The proximal advancement of the push rod 70 pushes the tab 64 on the gripper ring 150. This movement pivots the gripper ring 150 about the pivot axis 68 and engages the gripper ring 150 with the plunger 36 (shown in Fig. 2).

Once the gripper ring 150 has been engaged with the plunger 36, further proximal advancement of the push rod 70 moves the plunger 36 proximally, drawing air into the injection tube 38 to create the air pocket 42. This continues until the push rod 70 reaches the apex of the cam surface 74 at which point, further rotation of the face cam 72 causes the push rod 70 to withdraw distally. As the push rod 70 withdraws distally, a spring causes gripper ring 150 to return to its original orientation (i.e. substantially perpendicular to the longitudinal axis 126).

Referring now to Figs. 11 and 12, it can be seen that a plunger 236 having a tapered portion 80 can be used in combination with the air-in-tip mechanism to vary the amount of air that is drawn into the injection tube 38 (see Fig. 3) in proportion to the amount of fluid medicament that is initially present in the syringe 230. Specifically, for this purpose, the plunger 236 is formed with a tapered portion 80 having a decreasing diameter in the proximal direction, as shown. The consequence of the tapered portion 80 is best appreciated by comparing Fig. 11 to Fig. 12.

In greater detail, Fig. 11 shows the initial position of the plunger 236 (i.e. before retraction by the air-in-tip mechanism) when a relatively large volume of fluid is in the syringe 230, while Fig. 12 shows the initial position of the plunger 236 when a relatively small volume of fluid is in the syringe 230. Comparing Fig. 11 to Fig. 12, it can be seen that the addition of fluid to the syringe 230 causes the initial position of the plunger 236 to move proximally. Consequently, with more fluid in the syringe 230 (i.e. Fig. 11), a larger diameter portion of the plunger 236 is disposed within the aperture of the gripper ring 250 prior to activation of the air-in-tip mechanism.

From a comparison of Figs. 11 and 12, it can be further seen that a larger movement of the button 252 is required to tilt and thereby engage the gripper ring 250 with the small diameter portion of the plunger 236 disposed in the gripper ring 250 (Fig. 12) than is required to tilt and engage the gripper ring 250 with the large diameter portion of the plunger 236 disposed in the gripper ring 250 (Fig. 11). It is to be appreciated that the button 252 moves through the same total distance in the proximal direction regardless of the amount of fluid in the syringe 230. Thus, with more fluid in the syringe 230 (Fig. 11), only a relatively small amount of button 252 movement is expended engaging the plunger 236, leaving a relatively large amount of button 252 movement to retract the plunger 236. On the other hand, with less fluid in the syringe 230 (Fig. 12), a relatively large amount of button 252 movement is expended to engage the plunger 236, leaving only a relatively small amount of button 252 movement to retract the plunger 236. The consequence of this is that the plunger 236 will be retracted farther when more fluid is in the syringe 230, resulting in a larger air pocket 42 (see Fig. 3).

It is to be appreciated by those skilled in the pertinent art that the tapered plunger 236 can also be used in the embodiment of the air-in-tip mechanism shown in Fig. 8 to vary the size of the air pocket 42 (see Fig. 3) in proportion to the amount of fluid medicament that is initially present in the syringe 230.

While the particular Jet Injectors Having a Mechanism for Creating Air-In-Tip as herein shown and disclosed in detail is fully capable of obtaining the objects and providing the advantages herein before stated, it is to be understood that it is merely illustrative of the presently preferred embodiments of the invention and that no limitations are intended to the details of construction or design herein shown other than as described in the appended claims.

## Claims

1. A device for retracting a plunger through a fluid-filled chamber of a syringe to draw a predetermined amount of air into the syringe, said device comprising:
a base member engageable with the syringe, said base member defining an axis;
a gripper having an aperture, said gripper being mounted on said base member and centered on said axis to receive the plunger through said aperture;
a means mounted on said base member for selectively moving said gripper between a first orientation wherein said gripper is substantially perpendicular to said axis to allow an axial movement of the plunger through said aperture relative to said gripper, and a second orientation wherein said gripper is tilted relative to said axis to engage said gripper with the plunger to prevent an axial movement of the plunger through said aperture relative to said gripper; and
a means for retracting the plunger through a predetermined distance when said gripper is in said second orientation to draw the predetermined amount of air into the syringe.

2. A device as recited in claim 1 wherein said gripper is shaped as a ring and wherein said device further comprises a plunger guide mounted on said base member for aligning the plunger along said axis upon engagement of the syringe with said base member.

3. A device as recited in claim 2 further comprising a gripper return spring mounted on said base member to urge said gripper against said plunger guide with said gripper in said first orientation.

4. A device as recited in claim 1 wherein said retracting means is a button, said button being slideably mounted on said base member for movement through said predetermined distance between a first position and a second position.

5. A device as recited in claim 4 further comprising a means for adjusting said second position for said button to selectively alter the predetermined amount of air drawn into the nozzle section of the chamber.

6. A device as recited in claim 4 further comprising a button return spring mounted on said base member to urge said button into said first position.

7. A device as recited in claim 1 wherein said device further comprises a locking ring mounted on said base member for rotation about said axis to secure said syringe to said base member and said retracting means comprises a face cam having a cam surface, said face cam mounted on said locking ring, and a push rod having a first end resting on said cam surface and a second end in contact with said gripper to retract said plunger in response to the rotation of said locking ring to secure said syringe.

8. A device as recited in claim 7 further comprising a means for biasing said gripper into said first orientation.

9. A device for retracting a plunger through a fluid-filled chamber of a syringe to draw a predetermined amount of air into the syringe, said device comprising:
a base member for holding the syringe, said base member defining an axis;
a locking ring mounted on said base member for rotation about said axis to secure said syringe to said base member;
a face cam mounted on said locking ring for rotation therewith; and
a means responsive to a rotation of said face cam for engaging and retracting said plunger to draw the predetermined amount of air into the syringe.

10. A device as recited in claim 9 wherein said engaging and retracting means comprises a gripper ring having an aperture, said gripper ring being mounted on said base member and centered on said axis to receive the plunger through said aperture, said engaging and retracting means further comprising a push rod having a first end resting on said cam surface and a second end in contact with said gripper ring to tilt said gripper ring relative to said axis and engage said plunger with said gripper ring in response to a first rotation of said face cam and move said plunger through a predetermined distance to retract said plunger in response to a second rotation of said face cam.

11. A device as recited in claim 10 further comprising a means for biasing said gripper ring into an orientation wherein said gripper ring is substantially perpendicular to said axis.

12. A method for retracting a plunger through a fluid-filled chamber of a syringe to draw a predetermined amount of air into the syringe, said method comprising the steps of:
engaging a base member with the syringe, said base member defining an axis;
mounting a gripper ring having an aperture on said base member, said gripper ring being centered on said axis to receive the plunger through said aperture;
selectively moving said gripper ring between a first orientation wherein said gripper ring is substantially perpendicular to said axis to allow an axial movement of the plunger through said aperture relative to said gripper ring, and a second orientation wherein said gripper ring is tilted relative to said axis to engage said gripper ring with the plunger to prevent an axial movement of the plunger through said aperture relative to said gripper ring; and
retracting the plunger through a predetermined distance when said gripper ring is in said second orientation to draw the predetermined amount of air into the syringe.

13. A method as recited in claim 12 further comprising the step of mounting a plunger guide on said base member for aligning the plunger along said axis upon engagement of the syringe with said base member.

14. A method as recited in claim 13 further comprising the step of mounting a gripper return spring on said base member to urge said gripper ring against said plunger guide with said gripper ring in said first orientation.

15. A method as recited in claim 12 wherein said retracting step is accomplished using a button, said button being slideably mounted on said base member for movement through said predetermined distance between a first position and a second position.

16. A method as recited in claim 15 further comprising the step of mounting a button return spring on said base member to urge said button into said first position.

17. A method as recited in claim 12 further comprising the steps of:
mounting a locking ring on said base member for rotation about said axis;
mounting a face cam having a cam surface to said locking ring for rotation therewith;
positioning a first end of a push rod on said cam surface and a second end of said push rod in contact with said gripper ring;
turning said locking ring and said face cam through a first rotation to place said gripper ring in said second orientation; and
turning said locking ring and said face cam through a second rotation to retract the plunger through a predetermined distance and secure the syringe to said base member.

18. A method as recited in claim 17 further comprising the step of turning said locking ring and said face cam through a third rotation after the plunger has been retracted to move said gripper ring back to said first orientation from said second orientation to allow axial movement of the plunger through said aperture relative to said gripper ring during a subsequent injection.

19. A device for creating an air pocket in a syringe chamber with the air pocket having a volume proportion to the amount of fluid medicament in the syringe chamber, the syringe chamber having a distal end and a proximal end, said device comprising:
a plunger for insertion into said proximal end of said syringe chamber to expel fluid medicament from said distal end of said syringe chamber, said plunger being formed with a tapered section having a proximally decreasing diameter;
a base member engageable with the syringe, said base member defining an axis;
a gripper ring formed with an aperture and having a periphery, said gripper ring being mounted on said base member and centered on said axis to receive said tapered section of said plunger within said aperture; and
a means mounted on said base member for moving a portion of said periphery through a pre-determined distance, said distance having a first component for tilting said gripper ring relative to said axis to engage said gripper ring with said tapered section of said plunger, and a second component for moving said gripper ring and said plunger axially to create an air pocket in said syringe, wherein said second component of said distance increases with an increasing amount of fluid medicament in the syringe chamber to create an air pocket in the syringe chamber having a volume proportional to the amount of fluid medicament in the syringe chamber.

20. A device as recited in claim 19 wherein said moving means is a button, said button being slideably mounted on said base member and attached to said portion of said periphery of said gripper ring for movement through said predetermined distance.

21. A device as recited in claim 19 wherein said device further comprises a locking ring mounted on said base member for rotation about said axis to secure said syringe chamber to said base member and said moving means comprises a face cam having a cam surface, said face cam mounted on said locking ring for rotation therewith, and a push rod having a first end resting on said cam surface and a second end in contact with said portion of said periphery of said gripper ring to move said portion of said periphery of said gripper ring through said pre-determined distance in response to the rotation of said locking ring to secure said syringe chamber.
